# EUROPEAN PATENT SPECIFICATION

(11) **EP 1 124 847 B1**
(45) Date of publication and mention of the grant of the patent: **24.03.2004**
(21) Application number: 99951005.0
(22) Date of filing: 27.10.1999
(51) Int. Cl.: C07K 7/23, A61K 38/09, A61P 19/10

(54) **LHRH ANALOGUES FOR THE TREATMENT OF OSTEOPOROSIS**
LHRH-ANALOGA ZUR BEHANDLUNG DER OSTEOPOROSE
ANALOGUES DE LH-RH POUR LE TRAITEMENT DE L'OSTEOPOROSE

(30) Priority: 27.10.1998 GB 9823515
(43) Date of publication of application: 22.08.2001
(60) Divisional application: 03075482.4
(73) Proprietor: Ferring B.V., 2132 JX Hoofddorp (NL)
(72) Inventor: AKINSANYA, Karen, Bitterne Park, Southampton SO18 1GJ (GB); HAYWARD, Amanda, Cambridge CB4 1AW (GB); QI, Steve, Hedge End, Southampton SO30 4RS (GB)
(74) Representative: Geering, Keith Edwin
(86) International application number: PCT/GB1999/003553
(87) International publication number: WO 2000/024764

(56) References cited:
- WO-A-91/05563
- US-A- 4 540 513
- US-A- 4 721 775
- US-A- 5 091 367
- DATABASE MEDLINE [Online] US NATIONAL LIBRARY OF MEDICINE (NLM), BETHESDA, MD, US PAOLETTI A M ET AL.: "SPONTANEOUS REVERSIBILITY OF BONE LOSS INDUCED BY GONADOTROPIN - RELEASING HORMONE ANALOG TREATMENT" retrieved from STN XP002129776 & FERTILITY AND STERILITY (1996 APR) 65 (4) 707-10,

### SEQUENCE LISTING

INFORMATION FOR SEQ ID No 1.
   (i) Sequence Characteristics
      (a) Length: 19 bases
      (b) Type: nucleic acid
      (c) Strandedness: single
      (d) Topology: linear
   (ii) Molecule Type cDNA
   (iii) Sequence description: SEQ ID No. 1 CTG CAG CTG CCT GAA GGA G (1)
INFORMATION FOR SEQ ID No 2.
   (i) Sequence Characteristics
      (a) Length: 19 bases
      (b) Type: nucleic acid
      (c) Strandedness: single
      (d) Topology: linear
   (ii) Molecule Type cDNA
   (iii) Sequence description: SEQ ID No.2 GGG CGG GGC GGG GCT CTC G (2)
INFORMATION FOR SEQ ID No 3.
   (i) Sequence Characteristics
      (a) Length:.21 bases
      (b) Type: nucleic acid
      (c) Strandedness: single
      (d) Topology: linear
   (ii) Molecule Type cDNA
   (iii) Sequence description: SEQ ID No.3 ATT CTA CTG ACT TGG TGC GTG (3)
INFORMATION FOR SEQ ID No 4.
   (i) Sequence Characteristics
      (a) Length: 21 bases
      (b) Type: nucleic acid
      (c) Strandedness: single
      (d) Topology: linear
   (ii) Molecule Type cDNA
   (iii) Sequence description: SEQ ID No.4

GGA ATA TGT GCA ACT TGG TGT (4)

## Description

### FIELD OF INVENTION

The present invention relates to pharmaceutical compositions for the treatment of osteoporosis and other disorders of bone metabolism.

### BACKGROUND TO THE INVENTION

### i) Osteoporosis

Osteoporosis is a disease that affects many elderly people. Post-menopausal osteoporosis associated with oestrogen deficiency is the most common bone disease in the western world (Melton, J. Bone Miner. Res. 10 174-177, 1995). The basic mechanism underlying osteoporosis is when bone resorption exceeds bone formation resulting in net bone loss and an increase in the incidence of fractures. The dynamic processes that control bone morphogenesis take place in bone and bone marrow. Bone is a hard calcified connective tissue consisting of osteoprogenitor cells, osteoblasts, osteocytes and osteoclasts. Bone marrow is composed of haematopoietic stem cells and mesenchymal cells. These cells include osteoprogenitors, chondrocytes, adipocytes, fibroblasts and reticular cells. Bone metabolism and remodelling is dependent on a balance between two processes; bone resorption is dependent on osteoclast activity and bone formation is dependent on osteoblast activity. Osteoblast precursors are fibroblast-like, proliferating progenitor cells, which differentiate into osteoblasts that synthesise and deposit matrix to become new bone. Osteoclasts are a tissue specific subtype of macrophages responsible forresorption of bone. Mononuclear phagocytes from bone marrow fuse to form the large multinucleatedosteoclasts. Osteoclastogenesis is a complex phenomenon that is facilitated by the interaction of bone marrow stromal cells with haematopoietic osteoclast precursors (Suda *et al*., Endocr. Rev. 13 66-80, 1992).

A number of factors regulate the proliferation and differentiation of osteoblasts and osteoclasts including cell-cell interactions, cytokines, growth factors, steroid hormones and neurohormones. These factors act via endocrine, autocrine and paracine mechanisms. Parathyroid hormone (PTH) is a key systemic regulator of calcium and bone metabolism acting principally by regulating bone resorption. There is evidence to suggest that PTH, vitamin D and calcitonin actions may be mediated by local factors found in bone (Oursler *et al*., Endocrinol. 129 3313-3320, 1990). A number of growth factors have been implicated such as insulin-like growth factors (IGF-I and IGF-II), fibroblast growth factors FGF and aFGF) (Baylink *et al*., J. Bone Mineral Res. 8 (Supp, 2) S565-S572, 1993). Identification of locally produced agents that influence the process of bone formation and bone resorption may provide alternatives to current treatments for osteoporosis which include salmon calcitonin and hormone (oestrogen and progesterone) replacement therapy (Lindsay *et al*., Lancet 341 801-805, 1993).

### ii) GnRH-II

Studies on the physiology of the hypothalamic-pituitary-gonadal axis have resulted in the recognition of gonadotropin releasing hormone (GnRH, otherwise known as luteinizing hormone releasing hormone, LHRH) as a key regulatory hormone. GnRH is released by the hypothalamus and acts on the pituitary to stimulate the release ofluteinizing hormone (LH) and follicle-stimulating hormone (FSH). More recently, a peptide related toGnRH has been identified, first in chickens (Miyamato, US Patent No. 4,540,513) and subsequently in humans (White *et al*., Proc. Natl. Acad, Sci. USA 95 305-309, 1998). This peptide has been called GnRH-II. The sequences of the two peptides are compared below.

GnRH pyroGlu-His-Trp-Ser-Tyr-Gly-Leu-Arg-Pro-Gly-NH2 (5)

GnRH-II pyroGlu-His-Trp-Ser-His-Gly-Trp-Tyr-Pro-Gly-NH2 (6)

Analogues have also been reported (Volkers *et al*., US Patent No. 4,721,775). No clear evidence for a physiological role for GnRH-II has so far been presented.

### SUMMARY OF THE INVENTION

We have now found that GnRH-II is capable of modulating the differentiation of bone precursor cells and inducing the expansion of osteoblast populations.
As used herein, abbreviations referring to amino acids have their conventional meanings and indicate the natural L-isomer (except for the achiral amino acid glycine).

According to the present invention in a first aspect there is provided the use, for the preparation of a medicament for the treatment of bone metabolism disorder or for accelerating bone growth or repair, of a peptide according to the sequence pyroGlu-His-Trp-Ser-Xaa¹-Gly-Xaa²-Xaa³-Pro-Gly-NH₂     (7) or a salt thereof
wherein
**Xaa**^{**1**} is His or Tyr,
**Xaa**^{**2**} is Trp or Leu, and
**Xaa**^{**3**} is Tyr or Arg,
provided that when **Xaa**^{**1**} is Tyr and **Xaa**^{**2**} is Leu, then **Xaa**^{**3**} is not Arg.

Preferably, the medicament is for the treatment of osteoporosis, preferably age-related osteoporosis and osteoporosis associated with post-menopausal hormone status, primary and secondary hyperparathyroidism, disuse osteoporosis, diabetes-related osteoporosis, and glucocorticoid-related osteoporosis. The medicament may be for the treatment of other disorders of bone metabolism, or for accelerating bone growth or repair.

Preferably, **Xaa**^{**1**} is His, **Xaa**^{**2**} is Trp, and **Xaa**^{**3**} is Tyr. It will be recognised that such a peptide can form salts with acids (for example, acetic acid, trifluoroacetic acid, benzoic acid, hydrochloric acid, phosphoric acid and the like). To the extent that such salts are formed with pharmaceutically acceptable acids, they are included within the scope of the invention.

It will be understood by those familiar with the art that the medicament will also comprise one or more excipients such as diluents, carriers, preservatives, and the like. The medicament may be a solid or liquid formulation. For example, the medicament might be formulated as a tablet or capsule for oral administration, or as a suppository for rectal or vaginal use. Equally, the medicament might be formulated as a solution, for example in isotonic saline, for intravenous administration. The medicament may also be presented as a kit for formulation immediately prior to administration, for example as a freeze-fried powder in a sealed ampoule supplied with a second ampoule containing a solvent such as saline.

The medicament may be used in clinical situations other than for the treatment of osteoporosis, where bone mass or bone density are reduced, or where bone growth or repair needs to be accelerated. Such situations include (but are not limited to) the treatment of osteogenesis imperfecta and osteomalacia, the prevention of bone loss when an individual is immobilised for an extended period, bone segmental defects, periodontal disease, metastatic bone disease, osteolytic bone disease, other conditions requiring healing or regeneration of cartilage defects or injury, facial reconstruction procedures, and the facilitation of healing following a fracture. The compounds or medicaments of the present invention may also be used in conjunction with agents that affect bone resorption, for example antiresorptive agents, such as estrogen, bisphosphonates and calcitonin . In addition, or alternatively, the medicaments of the present invention may modulate calcium metabolism, cell proliferation and/or differentiation of normal or aberrant cells or tissues involved in bone physiology.

The preparation of the medicament or pharmaceutical composition may comprise the mixing of a peptide according to the sequence pyroGlu-His-Trp-Ser-Xaa¹-Gly-Xaa²-Xaa³-Pro-Gly-NH₂ (7) wherein **Xaa**^{**1**}**, Xaa**^{**2**} and **Xaa**^{**3**} are as defined above, with one or more pharmaceutically acceptable excipients. Mixing is taken to include the blending together of solids, the dissolution of one or more solids in a liquid, and the dispersion of a solid in a liquid. The method may also include such processes as freeze-drying and microencapsulation necessary to obtain the formulation to be presented. Such processes as are known in the art are included within the scope of the present invention.

The medicament may be for single administration, but is more likely to comprise a course of repeated administrations. The frequency of the administrations may be from once per month to four times per day. The amount of active peptide in each dose will depend on the dosing schedule and the route of administration. Generally, it will be between one microgram (1µg) and one hundred milligrams (100mg). Doses in the range 1-100µg are preferred when the composition is administered by intravenous injection. Doses in the range 10µg - 10mg are preferred when the composition is administered as slow-release depots. Doses in the range 1-100mg are preferred when the composition is administered orally. Furthermore, high doses and frequent administration will be preferred when the desired outcome is to increase bone mass while lower doses and less frequent administration are more suited to the maintenance of bone mass.

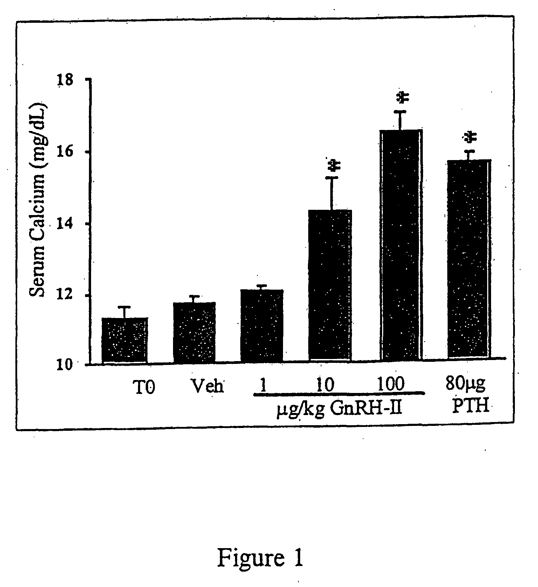

Figure 1 shows the effect of increasing doses of GnRH-II on serum calcium concentrations in ovariectomised rats.

The peptides that comprise the active agents of the medicaments of the present invention can be prepared by the methods generally known in the art. For example, the peptides may be prepared by solid-phase synthesis. This involves the sequential addition of amino acid residues to a resin-bound intermediate according to the following strategy.
1. Formation of resin-bound first intermediate
   PG-Aaa-OH + FG-Res → PG-Aaa-L-Res
   Aaa = amino acid
   PG = protecting group
   FG = functional group
   Res = polymeric resin
   L = linker group ( -O- or -NH- )
2. Deprotection
   PG-Aaa-L-Res → H-Aaa-L-Res
3. Chain extension
   PG-Bbb-OH + H-Aaa-L-Res → PG-Bbb-Aaa-L-Res
4. Repeat steps 2 and 3 as necessary
   PG-Bbb-Aaa-L-Res → → → PG-Nnn-...-Bbb-Aaa-L-Res
5. Cleave/deprotect
   PG-Nnn-...-Bbb-Aaa-L-Res → H-Nnn-...-Bbb-Aaa-OH (or -NH₂)

In step one, a protected amino acid is reacted with a functionalised resin. The protecting group (PG) is most commonly *tert*-butyloxycarbonyl (Boc) or 9-fluorenylmethyloxycarbonyl (Fmoc). The functional group on the resin (FG) is commonly a chloroalkyl group, a hydroxyl group or an amine group. When FG is a chloroalkyl or hydroxyl group, the linker group (L) is an oxygen atom ( -O- ). When FG is an amine group, L is -NH-.

In step two, the protecting group (PG) is removed from the α-amino group. When PG is Boc, this can be accomplished by treating the resin with acids such as trifluoroacetic acid or hydrogen chloride in dichloromethane. When PG is Fmoc, the deprotection can be accomplished by treating the resin with bases such as piperidine.

In step three, the peptide chain is extended by one amino acid residue. A protected amino acid is coupled to the amine group liberated in step two. Many reagents are known in the art for achieving this conversion. One combination is dicyclohexylcarbodiimide (DCC) and hydroxybenzotriazole (HOBt). Generally, a base will also be necessary. Suitable bases include triethylamine and N,N-diisopropylethylamine. The solvent will generally be dichloromethane, dimethylformamide, or a mixture of these.

If the side chains of the amino acids (Aaa - Nnn) contain reactive groups (for example amino groups, carboxylic acid groups, hydroxyl groups) then these will need protecting. The protecting groups chosen for the side chains are generally those that are stable under the conditions required to remove the protecting group (PG) from the α-amino group. If PG is Fmoc, then the side chain protecting groups can conveniently be based on tert-butyl chemistry. On the other hand, if PG is Boc, then the side chain protecting groups can be based on fluorenylmethyl chemistry. Other protecting groups known in the art can also be used.

In step four, the deprotection/chain extension cycle is repeated until the desired peptide sequence has been constructed.

In step five, the completed peptide is liberated from the resin. Protecting groups are removed from the side chains either before or after the cleavage. When L is -NH-, the peptide liberated is in the form of the C-terminal amide. When L is -O-, the peptide liberated is often the C-terminal free acid and a second step is required to form the C-terminal amide.

The peptides may also be prepared by solution-phase synthesis, and this may be more convenient when large quantities of material are needed.

The above general description is further elaborated below in a number of examples. These are intended to illustrate certain aspects of the invention. They are not intended to be limiting in any way.

### EXAMPLES

### Example 1 - Synthesis of GnRH-II

### 1A. Preparation of resin-bound protected peptide.

pyroGlu-His(Bom)-Trp(CHO)-Ser(Bzl)-His(Bom)-Gly-Trp(CHO)-Tyr(Bzl)-Pro-Gly- ORes

This peptide was prepared using standard solid-phase methods starting from Boc-Gly-esterified Merrifield resin (60 g, 1 mmol/g). The synthesis was performed in a manual synthesizer, with a total solvent and reagent volume of 300 mL for each operation. The standard deprotection/wash/coupling protocol is summarised in Table 1.

**Table 1**

| Step | Reagent | Time (min) | Number of operations |
|---|---|---|---|
| Deprotection of Boc | HCl / DCM* | 60 | 1 |
| Washing | DCM | 2 - 4 | 3 |
| Neutralisation | 10% DIPEA / DCM | 4 | 2 |
| Washing | DCM | 2 - 4 | 1 |
| Coupling | Activated ester | 60 - 120** | 1 - 2 |
| Washing | DCM | 2 - 4 | 3 |

| | | | |
|---|---|---|---|
| * Gaseous hydrogen chloride was bubbled through a suspension of the resin in DCM | | | |
| ** Completeness of reaction was determined by a negative ninhydrin test | | | |

Benzotriazolyl esters were used as the activated esters throughout the synthesis. These were prepared from the corresponding protected amino acids by reaction with 1-hydroxybenzotriazole (1eq.) and dicyclohexylcarbodiimide (1eq.). The quantities used (in relation to the resin substitution capacity) are listed in Table 2.

**Table 2**

| Cycle no. | Amino acid derivative | Molar excess |
|---|---|---|
| 1 | Boc-Pro-OH | 1.8 |
| 2 | Boc-Tyr(Bzl)-OH | 1.8 |
| 3 | Boc-Trp(CHO)-OH | 1.8 |
| 4 | Boc-Gly-OH | 1.8 |
| 5 | Boc-His(Bom)-OH | 1.8 |
| 6 | Boc-Ser(Bzl)-OH | 2.0 |
| 7 | Boc-Trp(CHO)-OH | 2.0 |
| 8 | Boc- His(Bom)-OH | 2.0 |
| 9 | pyroGlu-OH | 2.0 |

Following the final coupling, the resin was washed with dichloromethane (3 × 3L) and dried under reduced pressure at +40°C to constant weight.

Amino acid analysis: Consistent with proposed sequence

### 1 B. Cleavage and deprotection

pyroGlu-His-Trp-Ser-His-Gly-Trp-Tyr-Pro-Gly-NH2 (6)

The peptidoresin prepared in Example 1A was placed in a linen bag in a pressure vessel. The vessel was then charged with gaseous ammonia to a final pressure of 4.053 x 10⁵NM⁻²(4atm). After 72h the excess ammonia was vented and the resin was extracted with acetic acid (3×100mL) and ethanol (3×100mL). The combined extracts were degassed with nitrogen, 10% palladium-on-carbon was added, and the mixture was stirred under an atmosphere of hydrogen. When the reaction was complete (as judged by HPLC), the mixture was filtered and the filtrate was evaporated. The residue was purified by reverse-phase HPLC to give the title compound.

### Example 2 - Analysis of the effects of GnRH-II and analogues on Osteogenic cell populations in vitro.

(a) Human osteoblasts were isolated from cancerous bone from orthopaedic surgery (Nilsson *et al*., 1995) according to standard procedures known in the art. The bone explants were minced into small bone chips and then washed extensively in Dulbecco's modified Eagle's medium (DMEM)/F12 (1:1 Gibco, Paisley, U.K). These osteoblast like cells, Murine osteoblastic MC3T3-E1 cells and human clonal osteosarcoma cell lines MG-63 (non-mineralising) and SaOS-2 (mineralising osteosarcoma) were cultured in DMEM:F12, 1:1 with the addition of 10% fetal calf serum (FCS, Gibco), fungizone (500mg/l), gentamycin sulphate (50mg/l), L-glutamine (2mM) and I-ascorbic acid (100mg/l) in a humidified CQ chamber at 37°C.
(b) Human bone marrow stromal cells were isolated from bone fragments rinsed in phosphate-buffered saline. Bone marrow cells were collected and spun through a column of Ficoll Hypaque (Kimble *et al* J. Clin. Invest. 93 1959-1967, 1994). Cells at the interface were pelleted, counted and seeded into 75cm² flasks. The cells were incubated in a humidified CO₂ chamber at 37°C and the medium changed weekly. At confluence, the cells were harvested using trypsin EDTA and re-seeded in α-minimum essential medium (α-MEM) supplemented with 10% fetal calf serum (FCS, Gibco), penicillin (100U/ml), streptomycin (100mg/ml), fungizone and L-glutamine (2mM).
(c) All cells were serum-starved for 48h before addition of GnRH-I and GnRH-II. Cells were placed in DMEM without phenol red (in order to avoid oestrogen-like effects of phenol red) containing 10% charcoal-stripped serum for 48h in 12 well plates. Dose dependent effects of GnRH-I and GnRH-II and analogues of the peptides were studied following the addition of peptides at final concentrations ranging from 10⁹ to 10⁻⁶M. 1mM dibutyryl cAMP was used as a control. The cells were incubated for 24, 48 and 96h with the peptide being replaced every 24h.
(d) To assess the effects of the peptides on cell proliferation, [³H]thymidine was added at 1mCi/ml for an additional 24h and [³H]thymidine incorporation was determined. Radioisotope incorporation was determined using a scintillation counter and the results were calculated as cpm/mg of total protein.
(e) Expression of osteoblastic differentiation markers was also determined (Tintut Y *et al,.* J Biol Chem 273 7547-53, 1998). Total RNA was isolated at several stages before treatment, at 24, 48, 72 and 96h after addition of peptides. Type I procollagen, osteopontin and 28S RNA (used as an internal control) expression was determined by Northern blot analyses. Alkaline phosphatase, matrix GLA protein, osteoclastin and GAPDH (as an internal control) were determined by RT-PCR with specific primers designed for each gene.

The peptides of the invention caused significant effects at concentrations below 100µM.

### Example 3 - Analysis of the effects of GnRH-II and analogues on Osteoclast populations in vitro.

(a) Human clonal cell lines of osteoclast precursors (FLG 29.1) were used as an *in vitro* model of osteoclast differentiation (Gattei V *et al*., Cell Growth Differ 7 753-63, 1996). In addition, co-cultures of FLG 29.1 and osteoblastic cells (Saos-2) were evaluated for migratory, adhesive, cytochemical, morphological, and biochemical changes. Dose dependent effects of GnRH-I and GnRH-II and analogues of the peptides were studied following addition at final concentrations ranging from 10⁹ to 10⁻⁶M to FLG 29.1 cultures and to co-cultures. Parathyroid hormone was added as a control. Potentiation (or inhibition) of the differentiation of the preosteoclasts (fusion into large multinucleated elements) and a number of other factors were measured (Orlandini *et al,*. Cell Tissue Res. 281 33-42, 1995). These included:
   1. Positive staining for tartrate-resistant acid phosphatase in FLG 29.1 cells
   2. A decrease of the alkaline phosphatase activity expressed by Saos-2 cells
   3. The appearance of typical ultrastructural features of mature osteoclasts in FLG 29.1 cells
   4. 4.The release into the culture medium of granulocyte-macrophage colony stimulating factor.
   5. To assess the effects the peptides on cell proliferation, [³H]thymidine was added at 1mCi/ml for an additional 24h and [³H]thymidine incorporation was determined as described above.
(b) Bone marrow cells removed from human bone fragments were cultured in the presence of 10nM 1,25-(OH)₂ vitamin D₃ for seven days to generate multinucleated osteoclasts using standard techniques known in the art (Takahashi *et al*., Endocrinol 122 1473-1482, 1988). The culture medium (α-MEM) was removed and replaced by a fresh phenol red free medium supplemented with antibiotics and 10% charcoal-stripped heat-inactivated FCS containing GnRH-I, GnRH-II or analogues, and the cultures were maintained for a further 24h. Floating cells were harvested and osteoclasts stained for tartrate-resistant acid phosphatase (TRAP) expression, a marker of osteoclast differentiation (Hughes *et al*., Nat. Med. 2 1132-1135, 1996)
   1. Cells were incubated in 0.2M acetate buffer, pH 4.7-5.0, containing tartaric acid and 2% naphthol AS-BI phosphate (dissolved at 20mg/ml in ethylene glycolmonomethyl ether) for 15min at 37°C. The cells were then transferred to a second solution consisting of the same buffer and concentration of tartaric acid with 0.1% pararosanoiline chloride (hexazotised by mixing with an equal volume of 4% sodium nitrite for 5min at room temperature) for 10min at 37°C. This treatment causes a red cytoplasmic stain in cells expressing TRAP. Harris' hematoxylin was used as a nuclear counterstain.
   2. Apoptotic multinulceated osteoclasts were identified by strong expression of TRAP, larger size than accompanying viable TRAP-positive cells. Confirmation of apoptosis was carried out using acridine orange stain. Viable osteodasts were counted after fixation in 95% ethanol and TRAP hematoxytin staining, and apoptotic osteoclasts were expressed as a percentage of the total number of multinucleated osteoclasts (viable and apoptotic) in each culture well.

The peptides of the invention caused significant effects at concentrations below 100µM.

### Example 4- Expression analysis of GnRH mRNA in osteogenic and osteoclast cell populations

Total RNA was extracted from cells cultured as described above:
1. osteoblast like cells, isolated from cancerous bone
2. murine osteoblastic MC3T3-E1 cells
3. MG-63 (non-mineralising)
4. SaOS-2 (mineralising osteosarcoma)
5. human bone marrow stromal cells
6. human FLG 29.1 osteoclast precursor cells
7. multinucleated osteoclasts generated from bone marrow

Expression of GnRH-I and GnRH-II was determined by RT-PCR using PCR primers outlined in SEQ I.D. No 1-4. The integrity of the cDNA generated was determined by assessing the relative level of actin amplification.

### Example 5 - Effect of GnRH-II on bone mineral density in the ovariectomised rat

(a) Female adult (8 weeks old, 200-215g) Sprague Dawley rats were bilaterally ovariectomised (OVX). Animals were kept for 4 weeks post-delivery before commencing treatment. -Purina rat chow (1.00% calcium, 0.61% phosphorous) and water were provided ad libitum. Each study consisted of 6 weight-matched groups (n = 8/group).
(b) Treatment started 4 weeks post-OVX. After 4 weeks, a baseline control OVX group was sacrificed (Group A). The remaining groups were injected once a day with vehicle (Group B), 1µg/kg body weight (Group C), 10µg/kg body weight (Group D), 100µg/kg body weight (Group E) of GnRH-II, and 80µg/kg body weight (Group F) of hPTH(1-34).
(c) All rats were weighed every fourth day and dosages adjusted for 50g increase in mean group weight. Rats were given alternate subcutaneous injections ofcalcein (30mg/kg) or tetracyclin (30mg/kg) in 2% sodium bicarbonate-saline, respectively to label mineralization surfaces on days 10, 19 and 26, following treatment with drug. Bone mineral density was assessed by dual energy x-ray absorptometry-DEXA). On day 28 serum calcium levels were determined by colorimetric assay using a commercial kit.
(d) Success of OVX was confirmed at necropsy by failure to detect ovarian tissue and by observation of marked atrophy of the uterine horns. Both legs were disarticulated at the hip. The left tibia and femur were cleaned of excess muscle and soft tissue and placed in 70% ethanol. The anterior eminence of the right tibia metaphysis was shaved with a razor blade, barely exposing bone marrow. Both right femur and tibia were then placed in 10% phosphate-buffered formalin for 24h and transferred to 70% ethanol.

Ovariectomised animals treated daily with 10 and 100µg/kg of GnRH-II and 80µg/kg PTH for 28days have pronounced hypercalcemia. Results are shown in Figure 1.

### Example 6 - Cellular localisation of GnRH-II in paraffin sections of normal rat bone and human bone.

(a) Frozen and/or paraffin-embedded human and rat bone sections were fixed for 3-36h depending on size (3-5h at room temperature, then approx 24h at 4°C) and then soaked in 0.1M Tris + 5 % EDTA (12.11g + 50g EDTA) pH 7.3 until decalicified.
(b) Sections were then processed for antibody staining (rabbit polyclonal anti-GnRH-II antibody) using standard techniques.

Staining for GnRH-II was observed in platelets, megakaryocytes at the growth plate (especially proliferating chondrocytes). Some staining was also seen in the bone-forming cells particularly in active osetoblasts as well as new osteoid.

Overall, these results demonstrate that GnRH-II has a role in bone growth and calcium metabolism. Accordingly, it is useful as a therapeutic agent in diseases that are related to inadequate bone growth or loss of bone tissue.

## Claims

1. The use, for the preparation of a medicament for the treatment of bone metabolism disorder or for accelerating bone growth or repair, of a peptide according to the sequence pyroGlu-His-Trp-Ser-Xaa¹-Gly-Xaa²-Xaa³-Pro-Gly-NH₂          (7) or a salt thereof
wherein
**Xaa**^{**1**} is His or Tyr,
**Xaa**^{**2**} is Trp or Leu, and
**Xaa**^{**3**} is Tyr or Arg,
provided that when **Xaa**^{**1**} is Tyr and **Xaa**^{**2**} is Leu, then **Xaa**^{**3**} is not Arg.

2. A use according to claim 1 wherein the medicament is for the treatment of osteoporosis.

3. A use according to claim 2 wherein the medicament is for the treatment of age-related osteoporosis, osteoporosis associated with post-menopausal hormone status, primary or secondary hyperparathyroidism, disuse osteoporosis, diabetes-related osteoporosis, or glucocorticoid-related osteoporosis.

4. A use according to any preceding claim wherein the peptide sequence is

pyroGlu-His-Trp-Ser-His-Gly-Trp-Tyr-Pro-Gly-NH2 (6).

## Patentansprüche

1. Verwendung eines Peptids der folgenden Sequenz zur Herstellung eines Medikaments zur Behandlung von Knochenstoffwechselstörungen oder zur Beschleunigung von Knochenwachstum oder -reparatur: pyroGlu-His-Trp-Ser-Xaa^1-Gly-Xaa^2-Xaa^3-Pro-Gly-NH_2 (7) oder eines Salzes davon
wobei
Xaa¹ His oder Tyr ist,
Xaa² Trp oder Leu ist und
Xaa³ Tyr oder Arg ist,
vorausgesetzt, dass, wenn Xaa¹ Tyr und Xaa² Leu ist, Xaa³ nicht Arg ist.

2. Verwendung nach Anspruch 1, wobei das Medikament zur Behandlung von Osteoporose vorgesehen ist.

3. Verwendung nach Anspruch 2, wobei das Medikament zur Behandlung von altersbedingter Osteoporose, Osteoporose in Verbindung mit postmenopausalem Hormonstatus, Primär- oder Sekundärhyperparathyreoidismus, Inaktivitätsosteoporose, diabetesbedingter Osteoporose oder glukokortikoidbedingter Osteoporose vorgesehen ist.

4. Verwendung nach einem der vorherigen Ansprüche, wobei die Peptidsequenz wie folgt lautet:

pyroGlu-His-Trp-Ser-His-Gly-Trp-Tyr-Pro-Gly-NH2 (6).

## Revendications

1. Utilisation, pour préparer un médicament destiné au traitement d'un trouble du métabolisme osseux ou destiné à accélérer la croissance ou réparation osseuse, d'un peptide selon la séquence pyroGlu-His-Trp-Ser-Xaa1-Gly-Xaa2-Xaa3-Pro-Gly-NH2 (7) ou d'un sel de celui-ci
où
**Xaa**^{**1**} est His ou Tyr,
**Xaa**^{**2**} est Trp ou Leu, et
**Xaa**^{**3**} est Tyr ou Arg,
à condition que lorsque **Xaa**^{**1**} est Tyr et que **Xaa**^{**2**} est Leu, **Xaa**^{**3**} n'est alors pas Arg.

2. Utilisation selon la revendication 1, dans laquelle le médicament est destiné au traitement de l'ostéoporose.

3. Utilisation selon la revendication 2, dans laquelle le médicament est destiné au traitement de l'ostéoporose associée à l'âge, de l'ostéoporose associée à un état hormonal post-ménopausique, de l'hyperparathyroïdisme primaire ou secondaire, de l'ostéoporose d'immobilisation, de l'ostéoporose associée au diabète ou de l'ostéoporose associée au glucocorticoïdes.

4. Utilisation selon l'une quelconque des revendications précédentes dans laquelle la séquence peptidique est
